(19) Europäisches Patentamt
European Patent Office
Office européen des brevets

(11) Numéro de publication: **0 060 179**
**A1**

(12) # DEMANDE DE BREVET EUROPEEN

(21) Numéro de dépôt: **82400336.2**

(22) Date de dépôt: **26.02.82**

(51) Int. Cl.³: **C 07 D 211/70**
A 61 K 31/445

(30) Priorité: **11.03.81 FR 8104891**

(43) Date de publication de la demande: **15.09.82 Bulletin 82/37**

(84) Etats contractants désignés: **BE CH DE FR GB IT LI LU NL SE**

(71) Demandeur: **SANOFI, société anonyme**
**40, Avenue George V**
**F-75008 Paris(FR)**

(72) Inventeur: **Nisato, Dino**
**Viale Golgi 80**
**Pavia(IT)**

(72) Inventeur: **Bianchetti, Alberto**
**Via Corridoni 11**
**I-20122 Milan(IT)**

(72) Inventeur: **Pessa, Artemio**
**Piazzale Corvetto 3**
**I-20138 Milan(IT)**

(72) Inventeur: **Carminati, Paolo**
**Via Pacini 24**
**Milan(IT)**

(74) Mandataire: **Combe, André et al,**
**CABINET BEAU DE LOMENIE 55 rue d'Amsterdam**
**F-75008 Paris(FR)**

(54) **Compositions pharmaceutiques à action anorexigène.**

(57) Compositions pharmaceutiques renfermant, en tant qu'ingrédients actifs, des 4-(3-trifluorométhylphényl)-1,2,3,6-tétrahydropyridines de formule

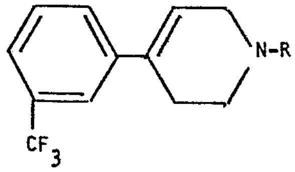

dans laquelle R représente de l'hydrogène, un groupe alkyle ou benzyle, ainsi que leurs sels, à activité anorexigène, utiles dans le traitement de l'obésité

EP 0 060 179 A1

Compositions pharmaceutiques à action anorexigène

La présente invention concerne des compositions pharmaceutiques renfermant des 1,2,3,6-tétrahydropyridines ou leurs sels utiles comme anorexigènes.

Plus particulièrement, l'invention se réfère à des compositions pharmaceutiques renfermant, en tant qu'ingrédients actifs, des 4-(3-trifluorométhylphényl)-1,2,3,6-tétrahydropyridines à activité anorexigène de formule

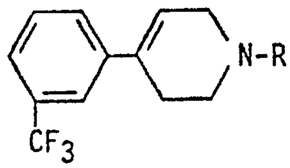

I

dans laquelle R représente de l'hydrogène, un groupe alkyle contenant de 1 à 6 atomes de carbone ou un groupe benzyle; ainsi que leurs sels pharmaceutiquement acceptables.

Il est connu que le chef de file des composés à action anorexigène est l'amphétamine qui exerce son activité par un mécanisme d'action biochimique central au niveau des systèmes dopaminergique et noradrénergique.

L'amphétamine et ses dérivés, tous caractérisés par une structure chimique comportant la séquence suivante

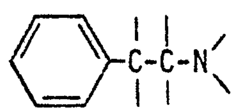

II

présentent des inconvénients importants car leur effet stimulant le système nerveux central ainsi que la possibilité d'accotumance et de pharmacodépendance peuvent constituer un danger potentiel pour le patient.

Les brevets français 1 459 013 et 4 949 M décrivent des composés de formule générale

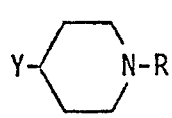

III

dans laquelle R est un radical amino, dialkylamino, cycloalkylméthyle,

alkyle, alkényle, alkynyle contenant chacun moins de 6 atomes de carbone et Y est un radical phényle substitué ou non, qui peuvent être utilisés comme agents analgésiques, comme agents provoquant une perte d'appétit ou comme agents actifs sur le système nerveux central. Selon ce qui est indiqué dans le brevet 4 949 M, les composés de formule III ci-dessus possèdent une propriété stimulante sympathomimétique centrale qui a été confirmée par un effet modéré, mais durable,de stimulation du système nerveux central chez des êtres humains volontaires.

Des études ont été donc consacrées à la recherche de dérivés de l'amphétamine présentant une dissociation entre l'activité stimulante et l'action anorexigène. L'introduction d'un groupe trifluorométhyle dans la position méta du groupe phényle de l'éthylamphétamine a donné lieu à l'obtention d'un produit, ci-après désigné par sa Dénomination Commune Internationale "fenfluramine",

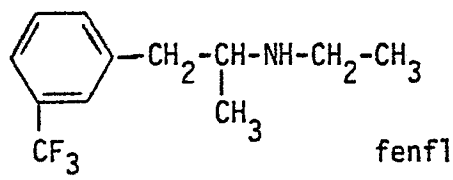

IV

fenfluramine

doté d'une excellente activité anorexigène, qui, au lieu d'être stimulant, possède une certaine action sédative.

Le brevet français 2 340 734 décrit une série de métatrifluorométhylphénylpipéridines à action anorexigène caractérisées par la formule générale suivante

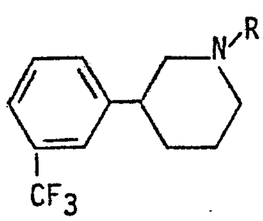

V

et, en tant que produit intermédiaire, un composé de formule

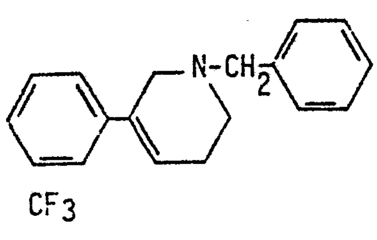

VI

Le brevet allemand publié au n. 2 904 826 décrit une série de métatrifluorométhylphényl-1,2,5,6-tétrahydropyridines à activité anorexigène ayant la formule générale suivante

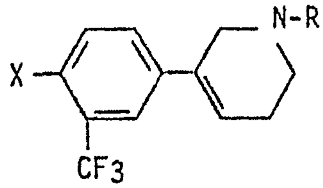

VII

dans laquelle X est un atome d'hydrogène ou de chlore et R représente un atome d'hydrogène ou un groupe alkyle, alkényle, alkynyle ou phénylalkyle et de laquelle le composé VI ci-dessus est exclu.

La fenfluramine et ses dérivés, par exemple les produits ayant les Dénominations Communes Internationales benfluorex, flucétorex, fludorex sont tous caractérisés par une structure chimique comportant la séquence suivante

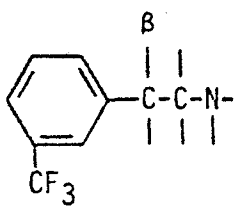

VIII

Les produits V, VI et VII ci-dessus peuvent également être considérés comme des dérivés de la fenfluramine car ils présentent la séquence VIII dans laquelle l'atome d'azote est lié à l'atome de carbone en position béta du radical métatrifluorométhylphénéthyle par un groupe propylène ou propanylidène de façon à former le noyau de la pipéridine ou de la 1,2,5,6-tétrahydropyridine.

L'avantage de la fenfluramine et de ses dérivés par rapport à l'amphétamine et à ses dérivés est dû au différent mécanisme d'action. En fait, l'anorexie provoquée par l'amphétamine semble être médiée par le "release" de noradrénaline cérébrale, alors que l'action anorexigène de la fenfluramine dépend du "release" de la sérotonine endogène des neurones centraux (Ann C. Sullivan et al. Appetite Regulation and Its Modulation by Drugs. NUTRITION AND DRUG INTERRELATION, 1978, Academic

Press, 21-82) et de l'inhibition de l'uptake de la sérotonine. Selon le brevet allemand cité ci-dessus, les composés de formule VII inhibent également l'uptake de sérotonine.

Il est cependant connu que la fenfluramine, à des doses très proches à la dose anorexigène, provoque une réduction significative des taux cérébraux de sérotonine (Arch. Intern. Pharmacodyn. Ther. 1967, 170, 276) et qu'une déplétion durable de sérotonine peut être considérée comme un signe de neurotoxicité potentielle (C.D. Morgan et al. Life Sci. Part I, 11, 83; 1972).

On a maintenant trouvé que les 4-(3-trifluorométhylphényl)-1,2,3,6-tétrahydropyridines de formule I ci-dessus, et leurs sels, montrent une activité anorexigène remarquable associée à une très basse toxicité. Sur le plan biochimique, les composés de formule I ci-dessus, ainsi que leurs sels, agissent en tant qu'agonistes de la sérotonine cérébrale sans provoquer aucune déplétion de sérotonine cérébrale ni stimulation du système nerveux central. Plus particulièrement, les composés de formule I ci-dessus montrent une grande affinité pour les récepteurs post-synaptiques de la sérotonine et, par cette stimulation directe du système sérotoninergique, ils entrainent une activité anorexigène sans les effets secondaires dûs au release de sérotonine.

L'activité anorexigène et les propriétés biochimiques des composés de formule I ci-dessus sont inattendues et surprenantes si l'on considère leur structure chimique qui présente la séquence

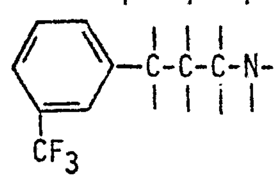

IX

tout à fait nouvelle pour un produit à action anorexigène avec un mécanisme d'action sérotoninergique et depourvu de toute action stimulante sur le système nerveux central.

Le brevet USA 3 125 488 décrit une série de 4-phényl-1,2,3,6-tétrahydropyridines avec une activité analgésique, spasmolytique et

sédative chez les animaux, lesdits produits ayant la formule

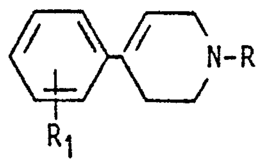

X

dans laquelle R est l'hydrogène, un alkyle inférieur tel que méthyle, éthyle, propyle et isopropyle, un groupe aralkyle, notamment benzyle et phénétyle, un alkényle inférieur tel que l'allyle, un alkynyle inférieur tel que le propargyle, un alkyle inférieur substitué par un groupe amino tel que diméthylaminoéthyle, et $R_1$ est l'hydrogène ou au moins un substituant tel qu'un halogène comme le chlore et le brome, un halogénoalkyle inférieur tel que chlorométhyle et trifluorométhyle, nitro, un alkyle inférieur tel que méthyle et éthyle, un alkoxy inférieur tel que méthoxy et éthoxy, un groupe carbalkoxy inférieur tel que carbométhoxy et carbéthoxy, ou un alkylènedioxy inférieur tel que le 3,4-méthylènedioxy; aussi bien comme bases libres que comme leurs sels.

Ledit brevet ne décrit par la 4-(3-trifluorométhylphényl)-1,2,3,6-tétrahydropyridine ni aucun de ses dérivés de substitution sur l'azote. Il ne suggère non plus la possbilité que les nouvelles 4-(3-trifluorométhylphényl)-1,2,3,6-tétrahydropyridines susbtituées possèdent une activité anorexigène remarquable avec un mécanisme d'action différent de celui des anorexigènes connus.

Ainsi la présente invention a pour objet des compositions pharmaceutiques renfermant, en tant qu'ingrédients actifs, les 4-(3-trifluorométhylphényl)-1,2,3,6-tétrahydropyridines de formule I ci-dessus, ainsi que leurs sels d'addition pharmaceutiquement acceptables.

Les sels pharmaceutiquement acceptables comprennent les sels non toxiques dérivés d'acides minéraux ou organiques tels que le chlorhydrate, le bromhydrate, le succinate, le tartrate, le citrate, le fumarate, le maléate, le 4,4'-méthylènebis-(3-hydroxy-2-naphtoate), ci-après désigné "pamoate", le 2-naphtalènesulfonate, ci-après désigné "napsylate", le méthanesulfonate, ci-après désigné "mésylate", le

p-toluènesulfonate, ci-après désigné "tosylate", et similaires.

La 4-(3-trifluorométhylphényl)-1,2,3,6-tétrahydropyridine est décrite dans les brevets français 1 421 208 et 1 459 013 comme intermédiaire dans la synthèse de produits à activité thérapeutique.

Ses dérivés N-alkylés sont inclus dans le brevet français 1 455 825 qui en indique une action vermifuge sans cependant envisager des compositions pharmaceutiques. Parmi lesdits dérivés N-alkylés, la 4-(3-trifluorométhylphényl)-1-méthyl-1,2,3,6-tétrahydropyridine est spécifiquement décrite.

Le dérivé N-benzyle, à savoir la 4-(3-trifluorométhylphényl)-1-benzyl-1,2,3,6-tétrahydropyridine, est inclus, sans cependant être décrit, dans la formule générale de certains produtis intermédiaires du brevet français 1 459 013 cité ci-dessus.

Les autres produits compris dans la formule générale I ci-dessus sont nouveaux.

Ils sont préparés en faisant réagir la 4-(3-trifluorométhylphényl)-1,2,3,6-tétrahydropyridine avec un dérivé halogéné R-X, où X représente un atome de chlore, de brome ou de iode, dans un solvant organique en présence d'un agent de condensation alcalin..

Comme solvant organique, on utilise un alcool, tel que méthanol, éthanol, n-butanol, n-pentanol ou bien de l'hexane, de la diméthylformamide, du diméthylsulfoxyde, du sulfolane, de l'acétonitrile, de la pyridine et similaires.

Les agents de condensation alcalins préférés sont les hydroxydes, les carbonates et le bicarbonates alcalins, mais des bases organiques telles que la triéthylamine ou la N-méthylpipéridine peuvent également être utilisées.

La température de réaction peut varier entre la température ambiante (environ 20°C) et 200°C et sa durée varie de conséquence. En général, après 4 à 5 heures de chauffage à 100-150°C, la réaction est complète et le produit final ainsi obtenu peut être isolé selon les

techniques conventionnelles et éventuellement transformé dans ses sels par traitement avec une solution de l'acide choisi dans un solvant organique.

Les 4-(3-trifluorométhylphényl)-1,2,3,6-tétrahydropyridines de formule I ci-dessus et leurs sels montrent une activité anorexigène remarquable et sélective sans donner aucun effet du type amphétaminique. La sélectivité de leur action est demontrée par la manque d'activités pharmacologiques secondaires; telles que l'activité sédative ou excitante et l'action inhibitrice de l'activité locomotrice.

L'activité anorexigène a été évaluée par la méthode de la prise de nourriture chez le rat. On a utilisé des rats femelles de 200 g entrainés pendant 10 jours à manger durant une période de 4 heures et sélectionnés au huitième jour. Au bout du dixième jour, les animaux randomisés ont été divisés en un "groupe contrôle", traité par le seul véhicule, et en plusieurs "groupes traités". Le traitement a été effectué par voie orale et/ou intrapéritonéale 30 minutes avant la présentation de la nourriture et, ensuite, on a mesuré la quantité de nourriture consommée au cours de la première heure.

Dans le tableau I sont reportés, pour cinq composés représentatifs utiles comme ingrédients actifs dans les compositions selon l'invention:

- la toxicité aiguë, exprimée comme $DL_{50}$ chez le rat par voie orale ou par voie intrapéritonéale (donnée A);
- l'activité anorexigène, exprimée comme dose orale ou intrapéritonéale inhibant de 50% la prise de nourriture ($DE_{50}$, donnée B);
- le rapport entre la toxicité aiguë et l'activité anorexigène qui exprime l'index thérapeutique lié à la toxicité aiguë (donnée A/B).

Comme produits représentatifs selon la présente invention on a utilisé les composés suivants:

- le chlorhydrate de 4-(3-trifluorométhylphényl)-1,2,3,6-tétrahydropyridine (ci-après indiquée par son numéro de code CM 57235),
- le chlorhydrate de 4-(3-trifluorométhylphényl)-1-benzyl-1,2,3,6-tétrahydropyridine (CM 57451),
- le chlorhydrate de 4-(3-trifluorométhylphényl)-1-propyl-1,2,3,6-tétrahydropyridine (CM 57545),
- le chlorhydrate de 4-(3-trifluorométhylphényl)-1-méthyl-1,2,3,6-tétrahydropyridine (CM 57548),
- le chlorhydrate de 4-(3-trifluorométhylphényl)-1-pentyl-1,2,3,6-tétrahydropyridine (CM 57756).

Comme produit de référence on a utilisé la fenfluramine.

TABLEAU I

| Produits | Voie d'administration | A | B | A/B |
|---|---|---|---|---|
| | | $DL_{50}$ mg/kg | $DE_{50}$ mg/kg | |
| CM 57235 | i.p. | 94,6 | 8,4 | 11,2 |
| | os | 346 | 15,7 | 22 |
| CM 57451 | i.p. | 220 | 15 | 14,6 |
| | os | 600 | 18 | 33,3 |
| CM 57545 | i.p. | 124 | 7 | 17,7 |
| | os | 178 | 8 | 22,2 |
| CM 57548 | i.p. | 185,7 | 12,2 | 15,2 |
| CM 57756 | os | 311,4 | 8,8 | 35,4 |
| fenfluramine | i.p. | 85,6 | 5,4 | 15,8 |
| | os | 100,4 | 4,0 | 25,1 |

De ce tableau il ressort que les produits représentatifs de la présente invention montrent une bonne activité anorexigène avec une faible toxicité. Leur efficatié est comparable, sur le plan de l'index thérapeutique, à celle du composé de référence.

Sur le plan biochimique, les 4-(3-trifluorométhylphényl)-1,2,3,6-tétrahydropyridines de la présente invention et leurs sels diffèrent de la fenfluramine et de ses dérivés dans leur mécanisme d'action. En fait, les composés de la présente invention sont des agonistes post-synaptiques de la sérotonine avec des faibles effets sur les mécanismes présynaptiques tels que l'uptake et le release de la sérotonine, sur lesquels, au contraire, la fenfluramine agit. Le mécanisme d'action des composés de la présente invention comporte une activité anorexigène remarquable et des effets secondaires réduits.

En particulier, les composés utilisés comme principes actifs des compositions pharmaceutiques de la présente invention, in vivo, ne provoquent pas de déplétion de sérotonine au niveau central. Le tableau II ci-dessous résume les taux cérébraux de sérotonine, en pourcentage par rapport aux contrôles, après administration orale ou intrapéritonéale de quatre ingrédients actifs des compositions pharmaceutiques de la présente invention. La détermination des taux cérébraux, selon Curzon et Green (Brit. J. Pharmacol. 39, 653, 1970), a été effectuée une heure et/ou deux heures après l'administration. La fenfluramine a été utilisée comme produit de référence.

TABLEAU II

| Composé | Voie d'administration | dose mg/kg | taux cérébraux de sérotonine % par rapport aux contrôles 1 h. | 2 h. |
|---|---|---|---|---|
| CM 57235 | i.p. | 15 | 106 | 102 |
| | | 7,5 | - | 112,0 |
| CM 57451 | i.p. | 15 | 121,2 | 112,0 |
| | | 30 | - | 119,0 |
| CM 57545 | i.p. | 7,5 | - | 103 |
| | | 15,0 | - | 112 |
| CM 57756 | os | 7,5 | - | 138 ** |
| | | 15 | - | 125 * |

suite TABLEAU II

| . Composé | Voie d'administration | dose mg/kg | taux cérébraux de sérotonine % par rapport aux contrôles 1 h. | 2 h. |
|---|---|---|---|---|
| fenfluramine | i.p. | 5,0 | 82,9 * | - |
| | | 7,5 | 81,6 * | 53,1 ** |

** significatif $P<0,01$  * significatif $P<0,05$
- non déterminé

De ce tableau il ressort que les produits de la présente invention, à des doses anorexigènes, ne diminuent pas les taux cérébraux de sérotonine, tandis que la fenfluramine, à des doses équivalentes, provoque une réduction importante de sérotonine cérébrale. Il y a donc une moindre possibilité que l'usage prolongé des composés de formule I provoque des effets secondaires au niveau central.

L'affinité des composés de la présente invention pour les récepteurs post-synaptiques de la sérotonine a été déterminée selon la méthode de Peroutka et Snyder (Molec. Pharmacol. 1979, 16, 687-699) qui consiste à incuber des membranes de cortex de rat avec une concentration fixée de $^3H$-sérotonine en présence de différentes concentrations de produit. Le tableau III montre la concentration molare de trois ingrédients actifs représentatifs de la présente invention qui donne une inhibition de 50% du binding spécifique au récepteur sérotoninergique ($IC_{50}$), à savoir la mesure de la capacité du produit d'intéragir avec la $^3H$-sérotonine dans le binding à son récepteur. La fenfluramine et la 3-(3-trifluorométhylphényl)-1-propyl-1,2,5,6-tétrahydropyridine (Composé A), décrite par le brevet allemand 2 904 826, ont été utilisées comme composés de référence.

TABLEAU III

| Composé | Binding $^3$H-sérotonine $IC_{50}$ /M/ |
|---|---|
| CM 57235 | $1{,}1 \cdot 10^{-7}$ |
| CM 57545 | $3{,}3 \cdot 10^{-7}$ |
| CM 57756 | $1{,}8 \cdot 10^{-7}$ |
| fenfluramine | $>10^{-5}$ |
| Composé A | $1{,}8 \cdot 10^{-6}$ |

De ce tableau il ressort que les composés de la présente invention ont une très bonne affinité pour le récepteur sérotoninergique post-synaptique alors que l'affinité des composés de référence pour le même récepteur est beaucoup plus faible.

Dans les compositions de la présente invention pour l'administration orale, sublinguale, sous-cutanée, intramusculaire, intraveineuse, transdermique ou rectale, les ingrédients actifs de formule I ci-dessus peuvent être administrés sous formes unitaires d'administration, en combinaison avec des supports pharmaceutiques classiques, aux animaux et aux êtres humains dans le traitement de l'obésité.

Parmi les formes unitaires d'administration appropriées, il y a les formes d'administration par voie orale telles que les comprimés, les gélules, les poudres, les granules et les solutions ou suspensions orales et les formes d'administration sublinguale, de même que les formes d'administration parentérale utiles pour une administration sous-cutanée, intramusculaire ou intraveineuse.

Afin d'obtenir l'effet anorexigène désiré, la dose de principe actif peut varier entre 0,1 et 100 mg par kg de poids du corps et par jour.

Chaque dose unitaire peut contenir de 1 à 500 mg d'ingrédient actif en combinaison avec un support pharmaceutique. Cette dose unitaire

peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de sucrose ou d'autres matières appropriées ou encore on peut les traiter d'une autre manière de telle sorte qu'ils aient une activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant acalorique, du méthylparaben et du propylparaben comme antiseptiques, ainsi qu'un agent donnant du goût et un colorant approprié.

Les poudres ou les granulats dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une application rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions particulières, stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à coté des 4-(3-trifluorométhylphényl)-1,2,3,6-tétrahydropyridines ou d'un de leurs sels pharmaceutiquement acceptables, d'autres principes actifs tels que, par exemple, des tranquillisants, des antidépresseurs, des hypolipémiants, des antidiabétiques ou d'autres médicaments qui peuvent être utilisés dans le traitement de l'obésité.

Les exemples suivants illustrent l'invention sans toutefois la limiter.

EXEMPLE 1

On chauffe au reflux pendant 5 heures un mélange de 5,67 g de 4-(3-trifluorométhylphényl)-1,2,3,6-tétrahydropyridine, 3,5 ml de triéthylamine et 3,15 g de 1-bromopropane dans 40 ml d'éthanol, puis on refroidit et on concentre. On reprend le résidu avec de l'éther diéthylique, on filtre, on lave à l'eau et l'on sèche. Par acidification avec une solution d'acide chlorhydrique en isopropanol, on obtient le chlorhydrate de 4-(3-trifluorométhylphényl)-1-propyl-1,2,3,6-tétrahydropyridine, désigné par son numéro de code CM 57545, qui, après cristallisation en isopropanol, fond à 198-200°C; rendement : 43,4%.

De la même façon, en faisant réagir la 4-(3-trifluorométhylphényl)-1,2,3,6-tétrahydropyridine avec le bromure de benzyle, le iodure de méthyle, le chlorure de néopentyle et, respectivement, le chlorure de n-pentyle, on obtient:

- le chlorhydrate de 4-(3-trifluorométhylphényl)-1-benzyl-1,2,3,6-tétrahydropyridine, désigné par son numéro de code CM 57451; p.f. 218 à 220°C;
- le chlorhydrate de 4-(3-trifluorométhylphényl)-1-méthyl-1,2,3,6-tétrahydropyridine, CM 57548; p.f. 243 à 246°C;
- le chlorhdyrate de 4-(3-trifluorométhylphényl)-1-néopentyl-1,2,3,6-tétrahydropyridine, CM 57601; p.f. 228 à 230°C; et, respectivement
- le chlorhydrate de 4-(3-trifluorométhylphényl)-1-n-pentyl-1,2,3,6-tétrahydropyridine, CM 57756; p.f. 209 à 211°C.

EXEMPLE 2

On ajoute une solution d'acide p-toluènesulfonique dans de l'acétone à une solution aqueuse de chlorhydrate de 4-(3-trifluorométhylphényl)-1,2,3,6-tétrahydropyridine, ci-après désigné par son numéro de code CM 57235.

Le produit qui précipite est isolé par filtration et séché.

On obtient ainsi le tosylate de 4-(3-trifluorométhylphényl)-1,2,3,6-tétrahydropyridine (numéro de code: CM 57710); p.f. 171-175°C.

De la même façon, par traitement d'une solution aqueuse de chlorhydrate de 4-(3-trifluorométhylphényl)-1,2,3,6-tétrahydropyridine avec une solution d'acide malique et, respectivement, d'acide maléique dans de l'acétone, on obtient:

le malate de 4-(3-trifluorométhylphényl)-1,2,3,6-tétrahydropyridine (numéro de code: CM 57711); p.f. 118-120°C, et, respectivement,

le maléate de 4-(3-trifluorométhylphényl)-1,2,3,6-tétrahydropyridine (numéro de code: CM 57713); p.f. 150-152°C.

D'une façon analogue, par traitement d'une solution aqueuse de chlorhydrate de 4-(3-trifluorométhylphényl)-1,2,3,6-tétrahydropyridine avec une solution aqueuse d'acide 2-naphtalènesulfonique, on obtient:

le napsylate de 4-(3-trifluorométhylphényl)-1,2,3,6-tétrahydropyridine (numéro de code: CM 57712); p.f. 180-183°C.

EXEMPLE 3

On prépare des gélules à base de chlorhydrate de 4-(3-trifluorométhylphényl)-1,2,3,6-tétrahydropyridine (CM 57235), ayant la composition suivante:

| | |
|---|---|
| CM 57235 | 15 mg |
| Lactose | 120 mg |
| Stéarate de magnésium | 5 mg |

en mélangeant intimement les ingrédients ci-dessus et en versant le

mélange dans de gélules de gélatine dure.

EXEMPLE 4

On prépare des comprimés ayant la composition suivante:

| | |
|---|---|
| CM 57235 | 20 mg |
| Lactose | 120 mg |
| Cellulose microcristalline | 30 mg |
| Amidon de maïs séché | 40 mg |
| Stéarate de magnésium | 5 mg |

en broyant l'ingrédient actif jusqu'à une dimension des particules de 0,4 mm, en le faisant passer à travers un tamis de 0,4 mm d'ouverture de maille, en mélangeant la matière broyée avec les autres constituants et en comprimant pour former les comprimés.

De la même façon on prépare des comprimés contenant 40 mg de CM 57235.

EXEMPLE 5

En opérant comme décrit dans l'Exemple 3, on prépare des comprimés ayant la composition suivante:

| | |
|---|---|
| CM 57235 | 50 mg |
| Lactose | 95 mg |
| Amidon de maïs | 100 mg |
| Talc | 4,5 mg |
| Stéarate de magnésium | 0,5 mg |

EXEMPLE 6

10.000 gélules avec une teneur en substance active de 50 mg sont préparées, à partir des constituants suivants: 500 g de chlorhydrate de 4-(3-trifluorométhylphényl)-1-propyl-1,2,3,6-tétrahydropyridine, 495 mg de cellulose microcristalline, 5 g de gel de silice amorphe. Les constituants ci-dessus sont bien mélangés et introduits dans des gélules de gélatine dure de dimension 4.

EXEMPLE 7

On prépare une solution aqueuse stérile appropriée pour une

utilisation parentérale en ampoules ayant la composition suivante:

| | | |
|---|---|---|
| Chlorhydrate de 4-(3-trifluoro-méthylphényl)-1,2,3,6-tétrahy-dropyridine | | 30 mg |
| Chlorure de sodium | | 5 mg |
| Eau distillée | q.s.p. | 2 ml |

EXEMPLE 8

On prépare des suppositoires ayant la composition suivante:

| | | |
|---|---|---|
| Chlorhydrate de 4-(3-trifluoro-méthylphényl)-1,2,3,6-tétrahy-dropyridine | | 50 mg |
| Lactose | | 250 mg |
| Witepsol W 45 | q.s.p. | 1,7 g |

On mélange la substance active avec le lactose et on met le mélange uniformement en suspension dans la masse pour suppositoires fondue. On verse la suspension dans des moules refroidis pour former des suppositoires d'un poids de 1,7 g.

De la même façon on prépare des suppositoires contenant:

50 mg de chlorhydrate de 4-(3-trifluorométhylphényl)-1-benzyl-1,2,3,6-tétrahydropyridine,

50 mg de chlorhydrate de 4-(3-trifluorométhylphényl)-1-méthyl-1,2,3,6-tétrahydropyridine, ou

50 mg de chlorhydrate de 4-(3-trifluorométhylphényl)-1-propyl-1,2,3,6-tétrahydropyridine.

EXEMPLE 9

On prépare des comprimés ayant la composition suivante:

| | |
|---|---|
| Chlorhydrate de 4-(3-trifluoro-méthylphényl)-1,2,3,6-tétrahy-dropyridine | 25 mg |
| Lactose | 95 mg |
| Amidon de maïs | 45 mg |
| Silice colloidale | 2 mg |

| | |
|---|---|
| Amidon soluble | 5 mg |
| Stéarate de magnésium | 3 mg |

On mélange la substance active avec une partie des excipients et on granule le mélange avec une solution de l'amidon soluble dans l'eau. Après séchage du granulat on ajoute le reste des excipients et on forme des comprimés par compression.

De la même façon, on prépare des comprimés contenant:

25 mg de chlorhydrate de 4-(3-trifluorméthylphényl)-1-méthyl-1,2,3,6-tétrahydropyridine,

25 mg de chlorhydrate de 4-(3-trifluorométhylphényl)-1-propyl-1,2,3,6-tétrahydropyridine,

25 mg de chlorhydrate de 4-(3-trifluorométhylphényl)-1-néopentyl-1,2,3,6-tétrahydropyridine, ou, respectivement,

25 mg de chlorhydrate de 4-(3-trifluorométhylphényl)-1-benzyl-1,2,3,6-tétrahydropyridine.

EXEMPLE 10

Selon le même mode opératoire décrit aux Exemples 3 à 9, en remplaçant l'ingrédient actif utilisé pour les compositions pharmaceutiques illustrées dans lesdits Exemples par les mêmes quantités de chlorhydrate de 4-(3-trifluorométhylphényl)-1-n-pentyl-1,2,3,6-tétrahydropyridine (CM 57756), on obtient, respectivement:

- des gélules contenant 15 mg de CM 57756,
- des comprimés contenant 20 ou 40 mg de CM 57756,
- des comprimés contenant 50 mg de CM 57756,
- des gélules contenant 50 mg de CM 57756,
- des ampoules contenant 30 mg de CM 57756,
- des suppositoires contenant 50 mg de CM 57756 et
- des comprimés contenant 25 mg de CM 57756.

REVENDICATIONS

1.Composition pharmaceutique à action anorexigène renfermant, en tant que principe actif,une 4-(3-trifluorométhylphényl)-1,2,3,6-tétrahydropyridine de formule

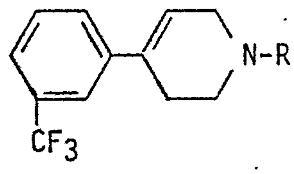

dans laquelle R représente de l'hydrogène, un groupe alkyle contenant de 1 à 6 atomes de carbone ou un groupe benzyle, ainsi qu'un de ses sels pharmaceutiquement acceptables.

Composition selon la revendication 1 sous forme d'unité de dosage contenant de 1 à 500 mg d'ingrédient actif par unité de dosage en mélange avec un excipient pharmaceutique.

2.Composition selon les revendication 1 et 2 caractérisée en ce qu'elle contient, en tant qu'ingrédient actif, le chlorhydrate de 4-(3-trifluorométhylphényl)-1,2,3,6-tétrahydropyridine.

3.Composition selon les revendications 1 et 2 caractérisée en ce qu'elle contient, en tant qu'ingrédient actif, le chlorhydrate de 4-(3-trifluorométhylphényl)-1-méthyl-1,2,3,6-tétrahydropyridine.

4.Composition selon les revendications 1 et 2 caractérisée en ce qu'elle contient, en tant qu'ingrédient actif, le chlorhydrate de 4-(3-trifluorométhylphényl)-1-propyl-1,2,3,6-tétrahydropyridine.

5.Composition selon les revendications 1 et 2 caractérisée en ce qu'elle contient, en tant qu'ingrédient actif, le chlorhydrate de 4-(3-trifluorométhylphényl)-1-n-pentyl-1,2,3,6-tétrahydropyridine.

6.Composition selon les revendications 1 et 2 caractérisée en ce qu'elle contient, en tant qu'ingrédient actif, le chlorhydrate de 4-(3-trifluorométhylphényl)-1-benzyl-1,2,3,6-trétrahydropyridine.

Office européen des brevets

**0060179**

Numéro de la demande

## RAPPORT DE RECHERCHE EUROPEENNE

EP 82 40 0336

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | DE-A-2 101 997 (BAYER)<br>* revendications *<br>--- | 1 | C 07 D 211/70<br>A 61 K 31/445 |
| Y | GB-A- 881 894 (PAUL ADRIAAN J. JANSSEN)<br>* pages 1,2 *<br>--- | 1 | |
| Y | FR-E- 86 403 (MAY AND BAKER)<br>* pages 1-2 *<br>--- | 1 | |
| D,X | US-A-3 284 457 (H. BESCHKE)<br>* colonnes 1-2 *<br>--- | 1,3 | |
| D,X | US-A-3 125 488 (J.H. BIEL et al.)<br>* colonnes 1-2 *<br>--- | 1,2 | DOMAINES TECHNIQUES RECHERCHES (Int. Cl. 3) |
| D,Y | FR-A-1 459 013 (ALLEN AND HANBURYS LTD.)<br>* pages 1-2 *<br>--- | 1 | C 07 D 211/00<br>A 61 K 31/00 |
| D,Y | FR-A-2 416 886 (ROUSSEL-VERLOF)<br>* revendications *<br>--- | 1 | |
| A | US-A-2 498 431 (J. LEE et al.)<br>--- | | |
| A | FR-A-2 070 167 (A.H. ROBINS CO.)<br>---<br>./. | | |

Le présent rapport de recherche a été établi pour toutes les revendications

| Lieu de la recherche | Date d'achèvement de la recherche | Examinateur |
|---|---|---|
| LA HAYE | 24-05-1982 | BRIGHENTI L.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

Office européen des brevets

# RAPPORT DE RECHERCHE EUROPEENNE

0060179

Numéro de la demande

EP 82 40 0336

Page 2

| DOCUMENTS CONSIDERES COMME PERTINENTS | | | | |
|---|---|---|---|---|
| Catégorie | Citation du document avec indication, en cas de besoin, des parties pertinentes | Revendication concernée | CLASSEMENT DE LA DEMANDE (Int. Cl. ³) | DOMAINES TECHNIQUES RECHERCHES (Int. Cl. ³) |
| A | US-A-3 833 576 (A. EDENHOFER)<br>----- | | | |

Le présent rapport de recherche a été établi pour toutes les revendications

| Lieu de la recherche | Date d'achèvement de la recherche | Examinateur |
|---|---|---|
| LA HAYE | 24-05-1982 | BRIGHENTI L.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82